# EUROPEAN PATENT SPECIFICATION

(11) **EP 3 797 730 B1**
(45) Date of publication and mention of the grant of the patent: **29.06.2022**
(21) Application number: 19807693.7
(22) Date of filing: 17.04.2019
(51) Int. Cl.: A61B 90/00, A61B 90/30, G06N 20/00, H05B 47/105, A61B 34/00

(54) **SURGERY INFORMATION PROCESSING DEVICE, INFORMATION PROCESSING METHOD, AND PROGRAM**
VORRICHTUNG ZUR VERARBEITUNG CHIRURGISCHER INFORMATIONEN, INFORMATIONSVERARBEITUNGSVERFAHREN UND PROGRAMM
DISPOSITIF DE TRAITEMENT D'INFORMATIONS DE CHIRURGIE, MÉTHODE DE TRAITEMENT D'INFORMATIONS, ET PROGRAMME

(30) Priority: 22.05.2018 JP 2018097551
(43) Date of publication of application: 31.03.2021
(73) Proprietor: Sony Group Corporation, Tokyo 108-0075 (JP)
(72) Inventor: TAKEMOTO, Masaya, Tokyo 108-0075 (JP); NAKAMURA, Yukihiro, Tokyo 108-0075 (JP); SHIRAKI, Hisakazu, Tokyo 108-0075 (JP); SUGIE, Yuki, Tokyo 108-0075 (JP)
(74) Representative: D Young & Co LLP
(86) International application number: PCT/JP2019/016411
(87) International publication number: WO 2019/225231

(56) References cited:
- WO-A1-2014/176403
- WO-A1-2016/154589
- WO-A1-2018/087941
- JP-A- 2008 535 202
- JP-A- 2012 023 492
- US-A1- 2009 261 759
- US-A1- 2014 346 957
- US-A1- 2015 208 478

## Description

### TECHNICAL FIELD

The present disclosure relates to a surgical information processing device, an information processing method, and a program.

### BACKGROUND ART

In surgery, an operative field is illuminated by an illumination device such as a surgical light so that an operator can easily perform the surgery. An illumination position and an illumination amount required by the operator change in accordance with progress of the surgery, and thus it is necessary to adjust an illumination state in the operative field.

For example, Patent Document 1 below discloses a method in which a driving state and a position state of a joint portion of an arm that supports an illumination device are detected, a position state of a chair on which a patient sits is detected, and an optimum illumination position is calculated on the basis of those states.

Patent Document 2 below discloses a teleoperated medical device including a sensor that senses information that represents a position of a control input out of an operator's field of view and displays the information to the operator.

Patent Document 3 below discloses a medical apparatus including a display optical system disposed within a display housing, the display optical system comprising a plurality of lens elements disposed along an optical path.

### CITATION LIST

### PATENT DOCUMENT

Patent Document 1: Japanese Patent Application Laid-Open No. 2004-129978
Patent Document 2: WO 2014/176403 A1
Patent Document 3: WO 2016/154589 A1

### SUMMARY OF THE INVENTION

### PROBLEMS TO BE SOLVED BY THE INVENTION

However, an operative field location varies depending on a patient in surgery, and furthermore, a location where illumination is required changes in real time depending on a progress status of the surgery. At this time, in order to adjust an illumination state of an illumination device, it is desired that not only an operator but also a person other than the operator, for example, a nurse, quickly grasp necessity of adjusting an illumination state in an operative field.

Therefore, in view of the above circumstances, the present disclosure proposes a surgical information processing device, an information processing method, and a program capable of quickly grasping necessity of adjusting an illumination state.

### SOLUTIONS TO PROBLEMS

Aspects of the present invention is set out in the appended set of claims.

### EFFECTS OF THE INVENTION

As described above, according to the present disclosure, a user can quickly grasp necessity of adjusting an illumination state in an operative field.

Note that the above effect is not necessarily limited, and in addition to the above effect, or in place of the above effect, any of the effects shown in the present specification or other effects that can be grasped from the present specification may be exhibited.

### BRIEF DESCRIPTION OF DRAWINGS

Fig. 1 is a block diagram showing a configuration of a surgical information processing system according to a first embodiment of the present disclosure.
Fig. 2 is a schematic view for explaining a presented display according to the same embodiment.
Fig. 3 is a flowchart for explaining operation of a surgical information processing device according to the same embodiment.
Fig. 4 is a block diagram showing a configuration of a surgical information processing system according to a second embodiment of the present disclosure.
Fig. 5 is a schematic view for explaining a presented display according to the same embodiment.
Fig. 6 is a flowchart for explaining operation of a surgical information processing device according to the same embodiment.
Fig. 7 is a flowchart for explaining a flow of machine learning for calculating an optimum illumination state in the surgical information processing device according to the same embodiment.
Fig. 8 is a flowchart for explaining a flow of machine learning for calculating the optimum illumination state in the surgical information processing device according to the same embodiment.
Fig. 9 is a schematic view for explaining a presented display according to the same embodiment.
Fig. 10 is a schematic view for explaining a presented display according to the same embodiment.
Fig. 11 is a schematic view for explaining a presented display according to the same embodiment.
Fig. 12 is a schematic view for explaining a presented display according to the same embodiment.
Fig. 13 is a schematic view for explaining a presented display according to a third embodiment of the present disclosure.
Fig. 14 is a flowchart for explaining operation of a surgical information processing device according to the same embodiment.
Fig. 15 is a diagram showing a hardware configuration example of the surgical information processing device according to the present disclosure.

### MODE FOR CARRYING OUT THE INVENTION

Hereinafter, preferred embodiments of the present disclosure will be described in detail with reference to the accompanying drawings. Note that, in the present specification and the drawings, components having substantially the same functional configuration are designated by the same reference numeral, and duplicate description thereof will be omitted.

Note that the description will be given in the following order.
<1. Background>
<2. First embodiment>
<3. Second embodiment>
<4. Third embodiment>
<5. Hardware configuration>
<6. Conclusion>

### <1. Background>

In surgery, a surgical light is used as an illumination device that illuminates an operative field so that an operator such as a surgeon can easily perform the surgery. Normally, since the surgical light is not sterilized, it is not preferable for the operator to touch and operate the surgical light. Therefore, in general, a nurse who assists the operator directly involved in the surgery, records the surgery, and adjusts surgical environment, etc. (hereinafter referred to as a perimeter nurse) physically operates an irradiation position, an irradiation angle, a diaphragm, and the like of the surgical light to provide the operator with a bright visual field. However, in addition to operating the surgical light, the perimeter nurse has various roles such as assisting the operator and recording the surgery as described above. Therefore, the perimeter nurse cannot always work near the surgical light. Furthermore, the perimeter nurse cannot always check brightness of the operative field by looking at the operative field. Therefore, depending on surgical development, when a surgical wound becomes deeper or a position of the operative field changes according to progress of the surgery, the perimeter nurse may be slow in noticing that the operative field has become dark, and the progress of the surgery may be delayed. If an operative site where the surgery is performed is in a dark state, attention and the like of the operator may be reduced. Furthermore, a skilled technique is required to operate the illumination device and irradiate the operative field with appropriate light. Therefore, even if the operator performing surgical operation operates a sterilized illumination device, it may take time to irradiate the operative field with appropriate light, and there is a possibility that the surgical operation is interrupted or delayed. In addition to this, there is a possibility that the attention of the operator is reduced, and there is a case where this reduction in attention of the operator affects the progress of the surgery.

In order to prevent such a delay in the progress of the surgery, reduction in attention of the surgeon, and the like, it is important for the perimeter nurse to operate the illumination device accurately in a short time to secure time for the other work. Therefore, the perimeter nurse is required to quickly determine whether or not an illumination state needs to be changed. Furthermore, it is important for the smooth progress of the surgery that the operator accurately tells the perimeter nurse a location where the operator wants to brighten.

Therefore, the present inventors have conceived of presenting a user such as an operator, a nurse, and the like with presentation based on an operative field image and illumination information indicating an illumination state of an illumination device, and resulted in invention of the present technology.

### <2. First embodiment>

### [2-1. Configuration]

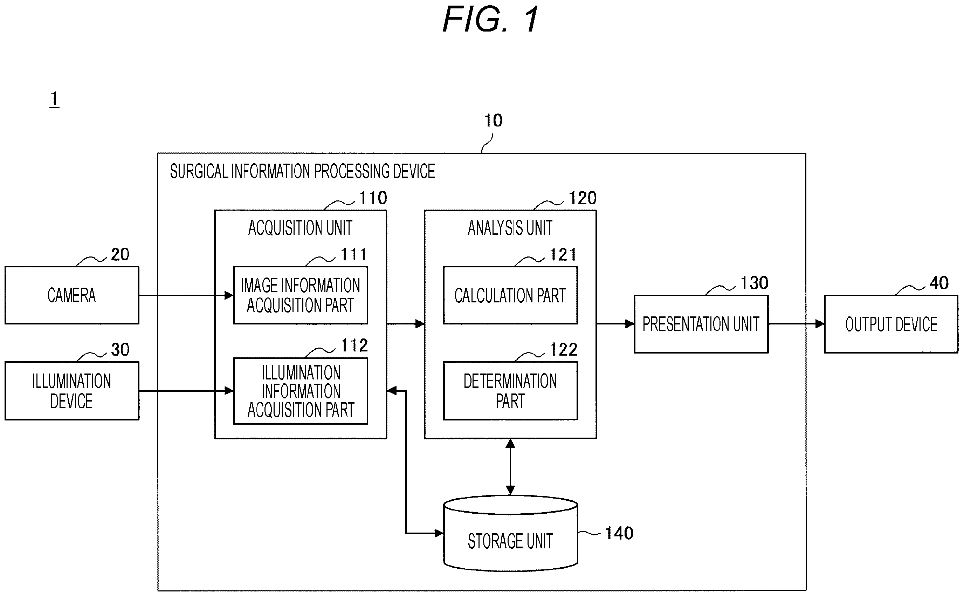

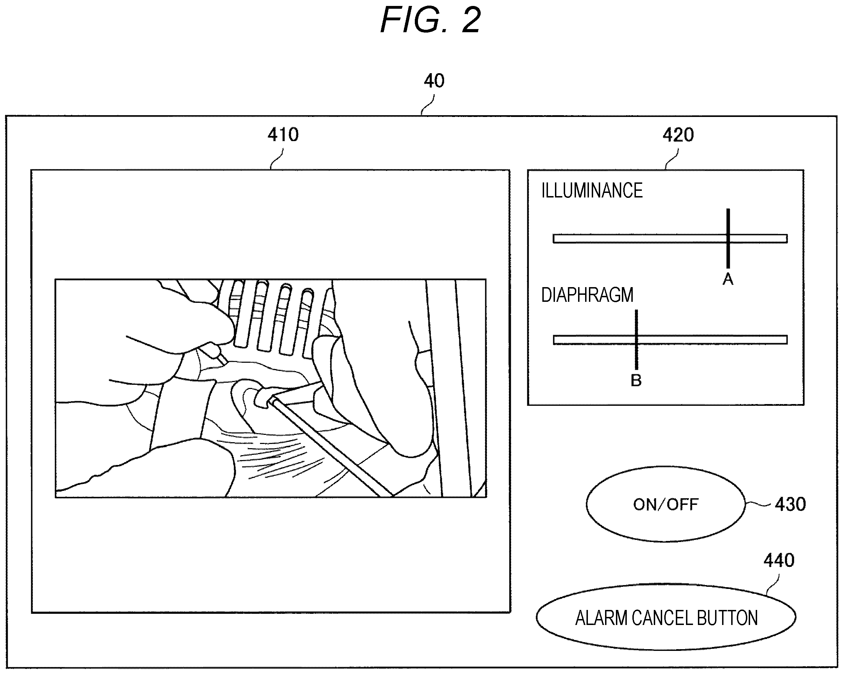

Up to this point, the background of inventing the present technology has been described. Next, a configuration of a surgical information processing device according to the present disclosure will be described with reference to Figs. 1 and 2.

First, a schematic configuration of a surgical information processing system 1 to which technology according to the present disclosure can be applied will be described. Fig. 1 is a diagram showing an example of a schematic configuration of the surgical information processing system 1 to which the technology according to the present disclosure can be applied. Fig. 2 is a schematic view for explaining a presented display according to the present embodiment.

The surgical information processing system 1 includes a surgical information processing device 10 according to the present embodiment, a camera 20, an illumination device 30, and an output device 40.

The surgical information processing device 10 according to the present embodiment acquires an operative field image in which an operative field illuminated by the illumination device 30 is imaged by the camera 20 and a set value of the illumination device 30, and performs presentation based on the operative field image and the set value of the illumination device 30 to a user. The set value of the illumination device 30 is a set value of an adjustment factor used for adjusting illumination light of the illumination device 30 and includes, for example, coordinate values indicating a position where the illumination device 30 is disposed, an illuminance value, a diaphragm value, a color temperature, and the like. Note that the surgical information processing device 10 according to the embodiment of the present disclosure can also carry out various processing while mutually cooperating with those having functions similar to the camera 20, the illumination device 30, and the output device 40, without cooperating with the camera 20, the illumination device 30, and the output device 40 in the surgical information processing system 1 as shown in Fig. 1.

The surgical information processing device 10 according to the present embodiment includes an acquisition unit 110, an analysis unit 120, a presentation unit 130, and a storage unit 140.

The acquisition unit 110 has a function of performing information communication with the camera 20 and the illumination device 30, which will be described later, via a network, and acquires an image captured by the camera 20 and a set value of the illumination device 30. Furthermore, the acquisition unit 110 may have a function of communicating information input to an external input device. The acquisition unit 110 may have a function of transmitting a control signal to the camera 20 and the illumination device 30 and controlling driving of the camera 20 and the illumination device 30. The control signal can include information regarding imaging conditions and illumination conditions, such as magnification and a focal length of the camera 20, and illumination intensity, a diaphragm, illumination intensity, and the like of the illumination device 30. The acquisition unit 110 includes an image information acquisition part 111 and an illumination information acquisition part 112.

The image information acquisition part 111 has a function of acquiring, from the camera 20, an operative field image captured by the camera 20 and operative field image information including setting information of the camera 20 when the operative field image has been captured. The operative field image information acquired by the image information acquisition part 111 includes, for example, brightness of the operative field image, color information forming the operative field image, a focal length, and the like. The operative field image information acquired by the image information acquisition part 111 is transmitted to the analysis unit 120.

The illumination information acquisition part 112 acquires, from the illumination device 30, a set value of the illumination device 30 when the operative field image has been acquired. (Hereinafter, "set value of the illumination device 30 when the operative field image has been acquired" is sometimes referred to as "current set value".) The current set value acquired by the illumination information acquisition part 112 is transmitted to the analysis unit 120. The set value of the illumination device 30 when this operative field image has been acquired corresponds to first illumination information.

The analysis unit 120 determines whether or not to present a user with setting of the illumination device 30 to be changed on the basis of the operative field image information acquired by the acquisition unit 110 and the set value of the illumination device 30 when the operative field image has been acquired. The analysis unit 120 includes a calculation part 121 and a determination part 122.

The calculation part 121 analyzes brightness distribution of the acquired operative field image, and with respect to the illumination device 30 that illuminates an operative site, calculates an optimum set value of the illumination device 30 for illuminating the operative site. (Hereinafter, "optimum set value of the illumination device 30 for illuminating an operative site" is sometimes referred to as "optimum set value".) This optimum set value of the illumination device 30 for illuminating the operative site corresponds to second illumination information. For example, a known image processing technique can be applied to the analysis of the brightness distribution. An analysis region of the brightness distribution of the operative field image performed by the calculation part 121 may be the entire operative field image, or may be a part of a region displayed in the operative field image, for example, brightness distribution of an operative site in which surgery is performed or a region designated by the user. Furthermore, the calculation of the optimum set value performed by the calculation part 121 may be performed by using a classifier generated by machine learning, for example. A known method may be applied to a machine learning method that can be applied to the present embodiment, and for example, a machine learning method using a neural network may be applied. In the machine learning method using the neural network, for example, a plurality of pieces of data, each of which has a classification label, is used as learning data. Parameters for classification are generated by inputting the learning data to a machine learning model using the neural network. Then, by inputting input data into the machine learning model in which the generated classification parameters are set, the input data is classified. At this time, it is preferable to output the classification and a likelihood of the classification. With such a machine learning method, even in a case where there is no information regarding factors that obstruct illumination of an operative field such as shadows caused by a user, surgical equipment, and the like, the calculation part 121 calculates an optimum set value by performing statistical processing.

Furthermore, the calculation part 121 may detect a surgical instrument displayed in the operative field image by a known surgical instrument detection technique and analyze brightness distribution of a region near a location where the surgical instrument has been detected. For example, a surgical instrument detection technique disclosed in Japanese Patent Application Laid-Open No. 2017-164007 or WO2015/037340 can be used for this surgical instrument detection. Furthermore, the calculation part 121 may detect a visual field position by applying a known visual field position estimation technique and analyze brightness distribution of a region including the visual field position. For example, a visual field position estimation technique disclosed in Japanese Patent Application Laid-Open No. 2016-182228 can be used for this visual field position estimation. The calculation part 121 may detect a portion pointed by a user using a laser and the like by using the known image processing technique and analyze brightness distribution of a region including the portion.

The determination part 122 compares a current set value and an optimum set value, and determines whether or not to perform presentation prompting a user to change setting of the illumination device 30. Specifically, a difference between the current set value and the optimum set value is calculated, and in a case where this difference is larger than a predetermined threshold value, the determination part 122 transmits a signal to change the setting of the illumination device 30 to the presentation unit 130. For example, regarding illuminance which is one set value, in a case where a difference between an illuminance value of the illumination device 30 when an operative field image has been acquired and an optimum illuminance value is larger than a predetermined threshold value, the determination part 122 transmits a signal to change the setting of the illumination device 30 to the presentation unit 130. Note that the determination part 122 may calculate a difference between set values for all adjustment factors of the illumination device 30, or may calculate a difference between set values for preselected adjustment factors. Furthermore, the above threshold value may be set by a user or may be set on the basis of a classifier generated by machine learning.

The presentation unit 130 has a function of presenting a notification prompting a change in setting conditions of the illumination device 30. Furthermore, the presentation unit 130 has a function of presenting, to a user, an operative field image and a set value of the illumination device 30 when the operative field image has been acquired. The presentation unit 130 controls information output by the output device 40 and an output method. For example, the presentation unit 130 transmits, to the output device 40, the notification prompting the change in the setting conditions of the illumination device 30, the image information acquired by the camera 20, the illumination information of the illumination device 30, and the like, and causes the output device 40 to output them. Furthermore, the presentation unit 130 may appropriately present a status regarding surgery (for example, elapsed time of the surgery, physical information of a patient, and the like) to the region.

The storage unit 140 appropriately records images acquired by the camera 20, various programs, databases, and the like used by the acquisition unit 110, the analysis unit 120, and the presentation unit 130 when performing the various processing as described above. Furthermore, the storage unit 140 may record various information acquired by the acquisition unit 110 as described above as history information. Moreover, the storage unit 140 may appropriately record, for example, various parameters, progress of processing, and the like that need to be saved when the calculation part 121 and the determination part 122 perform their processing. Furthermore, it is not limited to the processing executed by the calculation part 121 and the determination part 122. Various parameters, progress of processing, and the like that need to be saved when the surgical information processing device 10 according to the present embodiment performs some processing may be appropriately recorded. The storage unit 140 can be freely read/written by the acquisition unit 110 and the analysis unit 120.

The camera 20 is a medical observation device having a function of imaging an operative field in which surgery is performed and acquiring an operative field image. The camera 20 is, for example, a surgical video microscope or an operative field camera. The camera 20 may be provided with a magnification adjustment function, a focal length adjustment function, an imaging direction adjustment function, and the like. The camera 20 is not particularly limited as long as it can image an operative field under a surgical light used as the illumination device 30. For example, the camera 20 may be integrated with the illumination device 30.

For example, a complementary metal oxide semiconductor (CMOS) type image sensor having a Bayer array and capable of color imaging is used as an imaging element of the camera 20. Note that, for example, an element capable of capturing a high-resolution image of 4K or higher may be used as the imaging element. By obtaining an image of an operative site with high resolution, a user can grasp a state of the operative site in more detail, and surgery can be progressed more smoothly.

Furthermore, the imaging element of the camera 20 has a pair of imaging elements for acquiring image signals for a right eye and a left eye corresponding to a 3D display. The 3D display enables an operator to grasp a depth of a biological tissue in the operative site more accurately.

The camera 20 is provided with a communication unit (not shown), and transmits and receives various information to and from the acquisition unit 110. The communication unit of the camera 20 transmits the acquired image signal as RAW data to the acquisition unit 110. At this time, it is preferable that the image signal is transmitted by optical communication in order to display the captured image of the operative site with low latency.

Furthermore, the camera 20 has a function of receiving a control signal for controlling driving of the camera 20 from the acquisition unit 110. For example, the control signal includes information regarding imaging conditions, such as information that specifies a frame rate of a captured image, information that specifies an exposure value at the time of image capturing, and/or information that specifies magnification and a focus of the captured image.

Note that the imaging conditions such as the frame rate, the exposure value, the magnification, the focus, and the like described above are automatically set by the acquisition unit 110 on the basis of the acquired image signal. In other words, so-called auto exposure (AE) function, auto focus (AF) function, and auto white balance (AWB) function are installed in the camera 20.

The illumination device 30 has a function of irradiating an operative site with light. The illumination device 30 is provided on a ceiling of an operating room and irradiates at least an operator's hand. The illumination device 30 may be capable of appropriately adjusting an amount of irradiation light, a wavelength (color) of the irradiation light, an irradiation direction of the light, and the like. The illumination device 30 has, for example, a plurality of LED light sources as a light source. The illumination device 30 is provided with a movable arm, and a position of the light source is arbitrarily determined by operation of a user. It is preferable that the illumination device 30 be configured so that a focal depth and a light field diameter of the illumination light emitted from the illumination device 30 can be adjusted. The illumination device 30 includes, for example, a surgical light, and may include a plurality of surgical lights.

The output device 40 has a function of outputting an operative field image, assist information that is information for assisting a user in operation, a predetermined voice, and the like according to an instruction from the presentation unit 130 included in the surgical information processing device 10. The assist information includes information regarding a set value of the illumination device 30 when an operative field image has been acquired. A display screen of the output device 40 has, for example, an operative field image display area 410 for displaying the operative field image and an assist information display area 420 for displaying the assist information. For example, in Fig. 2, a real-time image acquired by the camera 20 is displayed in the operative field image display area 410. Furthermore, illuminance and a diaphragm of the illumination device 30 are displayed in the assist information display area 420 on the output device 40. The assist information display area 420 displays the assist information in a display method that allows a user to easily grasp a state of the illumination device 30. For example, an illuminance value and a diaphragm value of the illumination device 30 may be displayed in a meter display or numerical display. Furthermore, as shown in Fig. 2, the output device 40 may include a switch 430 for controlling ON/OFF of the illumination device 30 and an alarm cancel button 440 for canceling an alarm issued at the time of prompting a user to set the illumination device 30. By checking the operative field image and the illumination information of the illumination device 30 displayed on the output device 40, the user can quickly grasp whether or not the illumination device 30 needs to be adjusted.

The output device 40 displays an image based on an image signal subjected to image processing by the presentation unit 130 under the control of the presentation unit 130. For example, in a case where the camera 20 corresponds to high-resolution imaging such as 4K (horizontal pixel number 3840 x vertical pixel number 2160) or 8K (horizontal pixel number 7680 x vertical pixel number 4320), and/or in a case where it corresponds to a 3D display, a device capable of the high-resolution display and/or a device capable of the 3D display can be used correspondingly as the output device 40. In a case where the output device 40 corresponds to the high-resolution imaging such as 4K or 8K, more immersive feeling can be obtained by using the output device 40 having a size of 55 inches or more. Furthermore, a plurality of output devices 40 having different resolutions and sizes may be provided according to application.

Up to this point, the schematic configurations of the surgical information processing system 1 and the surgical information processing device 10 to which the technology according to the present disclosure can be applied have been described.

### [2-2. Operation]

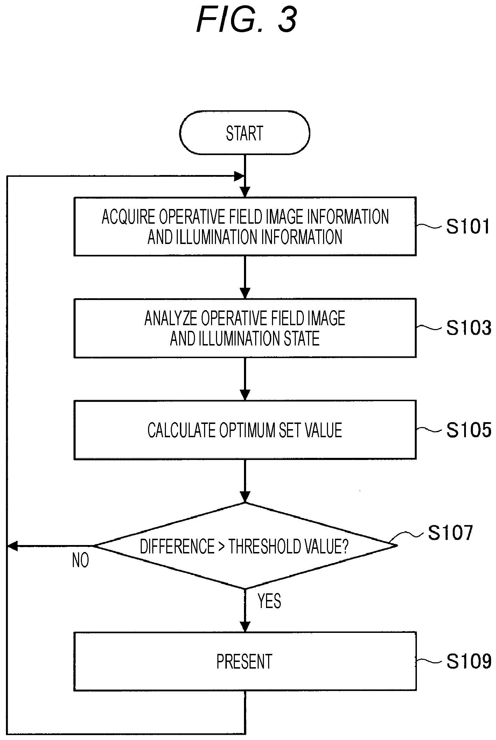

Next, operation of the surgical information processing device 10 according to the present embodiment will be described with reference to Fig. 3. Fig. 3 is a flowchart for explaining the operation of the surgical information processing device 10 according to the present embodiment.

First, the acquisition unit 110 acquires operative field image information acquired by the camera 20 and a set value of the illumination device 30 when the operative field image has been acquired (S101). Specifically, the image information acquisition part 111 included in the acquisition unit 110 acquires an operative field image captured by the camera 20 and image information including setting information of the camera 20 when the operative field image has been captured. At this time, the image information acquisition part 111 detects an operative site to be treated by applying, for example, a known surgical instrument detection technique, visual field position estimation technique, or the like. Therefore, the operative site that a user wants to brighten in the operative field image is detected. The acquired operative field image, operative field image information, and illumination information are transmitted to the analysis unit 120.

Subsequently, the calculation part 121 included in the analysis unit 120 analyzes the operative field image and an illumination state (S103). Specifically, the calculation part 121 analyzes the operative field image and calculates brightness distribution of an operative field. At this time, the calculation part 121 may analyze brightness distribution of the entire operative field or may analyze brightness distribution of a part of a display region of the operative field image. Specifically, the calculation part 121 may analyze brightness distribution of a region near a surgical instrument, may analyze brightness distribution of a region including a visual field position by detecting the visual field position, or may analyze brightness distribution of a region designated by a user.

Next, the calculation part 121 calculates an optimum set value of the illumination device 30 on the basis of the acquired image information and the brightness distribution (S105). Specifically, the calculation part 121 analyzes an operative field image that should be adjusted by the illumination device 30 and calculates an optimum set value for an operative site by using a classifier made to learn by machine learning.

Thereafter, the determination part 122 included in the analysis unit 120 compares a current set value and the optimum set value, and determines whether or not to perform presentation prompting a user to change setting of the illumination device 30 (S107). Specifically, the determination part 122 calculates a difference between the current set value and the optimum set value, compares this difference and a predetermined threshold value, and in a case where this difference is larger than the predetermined threshold value, the determination part 122 transmits information including the operative field image and the current set value to the presentation unit 130 (S107/YES). On the other hand, in a case where the above difference is equal to or less than the predetermined threshold value, the operation is repeatedly repeated from S101 (S107/NO).

Then, after receiving the information regarding the operative field image and the current set value from the determination part 122, the presentation unit 130 transmits, to the output device 40, a signal for prompting the user to operate the illumination device 30. Then, the output device 40 performs an output prompting operation of the illumination device 30 (S109). The output prompting the operation of the illumination device 30 may be performed by, for example, issuing a warning sound. The presentation unit 130 may transmit information of the above operative field image and the set value of the illumination device 30 when the operative field image has been acquired to the output device, and the output device 40 may output the received information of the operative field image and the set value. With the above operation, the user can quickly grasp necessity of adjusting the illumination state in the operative field.

### <3. Second embodiment>

### [3-1. Configuration]

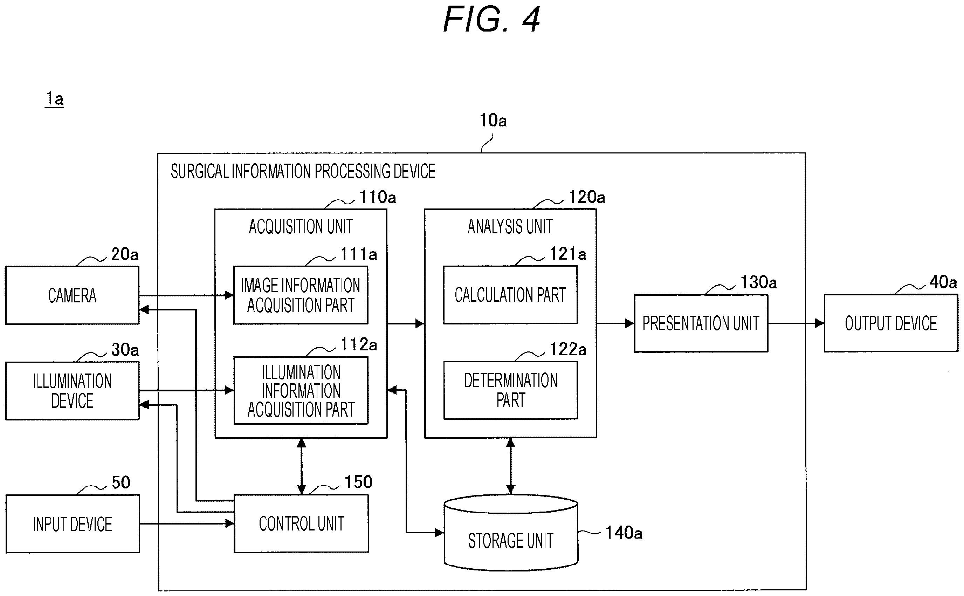

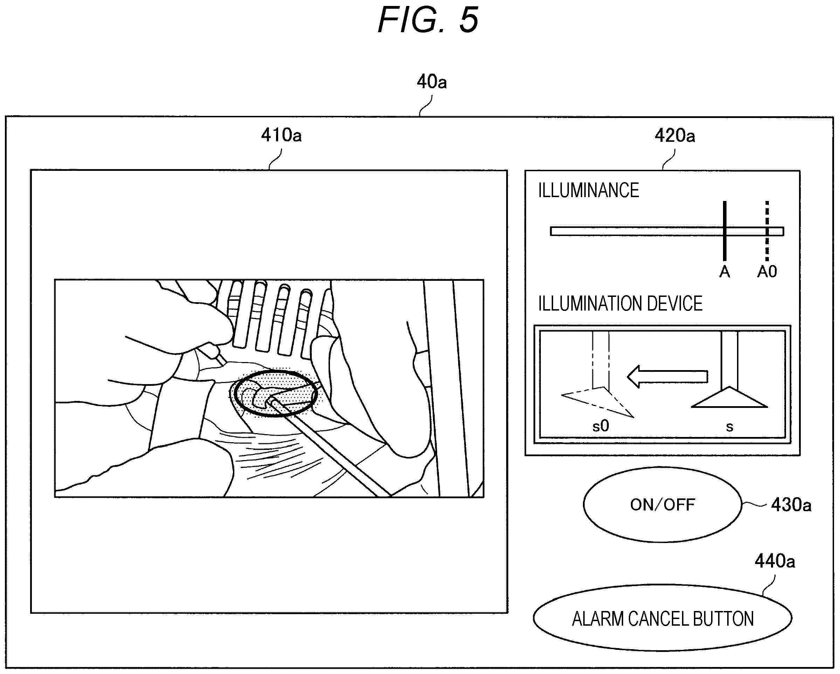

Subsequently, a second embodiment according to the present disclosure will be described with reference to Figs. 4 and 5. Fig. 4 is a diagram showing an example of a schematic configuration of a surgical information processing system 1a to which technology according to the present embodiment can be applied. Fig. 5 is a schematic view for explaining a presented display according to the present embodiment. The surgical information processing system 1a to which the technology according to the present embodiment can be applied includes a surgical information processing device 10a according to the present embodiment, a camera 20a, an illumination device 30a, an output device 40a, and an input device 50. Since the camera 20a, the illumination device 30a, and the output device 40a are similar to those described in the first embodiment, detailed description thereof will be omitted here. The surgical information processing system 1a to which the technology according to the present embodiment can be applied further includes the input device 50 in addition to the surgical information processing system 1 according to the first embodiment. The surgical information processing device 10a according to the present embodiment can perform various processing while mutually cooperating with the camera 20a, the illumination device 30a, the output device 40a, and the input device 50 in the surgical information processing system 1a as shown in Fig. 4. Furthermore, the surgical information processing device 10a according to the present embodiment can also perform various processing while mutually cooperating with those having functions similar to the camera 20a, the illumination device 30a, the output device 40a, and the input device 50, without cooperating with the camera 20a, the illumination device 30a, the output device 40a, and the input device 50 in the surgical information processing system 1a as shown in Fig. 4.

The surgical information processing device 10a includes an acquisition unit 110a, an analysis unit 120a, a presentation unit 130a, a storage unit 140a, and a control unit 150. Since the storage unit 140a is similar to that described in the first embodiment, detailed description thereof will be omitted here.

The acquisition unit 110a according to the present embodiment has a function of registering reference data when a classifier used for machine learning as described later is created, in addition to the function of the determination part 122 according to the first embodiment.

The analysis unit 120a according to the present embodiment includes a calculation part 121a and a determination part 122a. The determination part 122a determines whether or not a dark part, which is a dark region, exists in an operative field displayed in an operative field image, in addition to the function of the determination part 122 according to the first embodiment. In a case where the determination part 122a determines that the dark part exists in the operative field, the calculation part 121a calculates an optimum set value of the illumination device 30a. The presence/absence of the dark part is determined by using brightness distribution of a region displayed in an operative field image calculated by the calculation part 121a. Specifically, in a case where a region having brightness equal to or lower than a predetermined threshold value exists in the region displayed in the operative field image, the determination part 122a determines that the dark part exists. Furthermore, the determination part 122a has a function of registering teacher data when the classifier used for machine learning as described later is created.

The presentation unit 130a has a function of presenting an optimum set value of the illumination device 30a and a display regarding a dark part in an operative field, in addition to the function of the presentation unit 130 according to the first embodiment. Furthermore, in a case where the determination part 122a determines that the dark part exists in the operative field, the presentation unit 130a presents a notification prompting a change in setting conditions of the illumination device 30a. Therefore, a user can appropriately change setting of the illumination device 30a at appropriate timing.

The control unit 150 according to the present embodiment has a function of controlling the illumination device 30a. The control unit 150 is configured by a central processing unit (CPU), a graphics processing unit (GPU), and the like. The control unit 150 receives, for example, information input by the input device 50, and controls a set value of the illumination device 30a according to the received information. The control unit 150 may send a control signal to the camera 20a and control driving thereof. The control signal can include information regarding imaging conditions such as magnification and a focal length. The information regarding the imaging conditions can be input via the input device 50.

An output screen of the output device 40a includes an operative field image display area 410a, an assist information display area 420a, a switch 430a, and an alarm cancel button 440a. As shown in Fig. 5, the output device 40a has a function of superimposing a display showing a dark part on a real-time image displayed in the operative field image display area 410a, in addition to the function of the output device 40 according to the first embodiment. Furthermore, the output device 40a has a function of displaying a current set value and an optimum set value of the illumination device 30a in the assist information display area 420a. For example, in the assist information display area 420a, the optimum set value of the illumination device 30a calculated by the calculation part 121a is displayed in addition to real-time illuminance and a diaphragm of the illumination device 30a included in the current set value. Specifically, in Fig. 5, the assist information display area 420a displays real-time illuminance A and illuminance A0 that is an optimum set value for the dark part shown in the operative field image display area 410a. Furthermore, the assist information display area 420a displays real-time arrangement S of the illumination device 30 and arrangement S0 of the illumination device 30 in an optimum illumination state. A position of the dark part and the current set value and the optimum set value of the illumination device 30a are displayed on the output screen of the output device 40a, so that a user can visually grasp room for improvement of illumination. The user can quickly determine how to change setting of the illumination device 30a by checking the room for improvement of the illumination shown on the output device 40a. Note that the assist information displayed in the assist information display area 420a may be displayed according to setting conditions of the illumination device 30a that should be adjusted. Furthermore, setting conditions of the device selected by the user in advance may be displayed as the assist information. Examples of the output device 40a include a monitor provided in an operating room, an information processing terminal, a user interface (UI) of an OR system, and the like. Examples of the information processing terminal include a tablet, a PC, and the like. A device having a voice input function may be used as the output device 40a. The switch 430a has a function of controlling ON/OFF of the illumination device 30a, and the alarm cancel button 440a has a function of canceling an alarm issued at the time of prompting the user to set the illumination device 30a.

The input device 50 is operated by a user, and various information and instructions can be input to the surgical information processing device 10a. For example, the user inputs various information regarding surgery, such as physical information of a patient and information regarding a surgical procedure, through the input device 50. Furthermore, for example, the user inputs an instruction to change imaging conditions (a type of irradiation light, magnification, a focal length, and the like) by the camera 20a, an instruction to change a set value of the illumination device 30a, and the like.

The type of the input device 50 is not limited, and the input device 50 may be various known input devices. For example, a mouse, a keyboard, a touch panel, a switch, a foot switch 5171, and/or a lever, and the like can be used as the input device 50. In a case where the touch panel is used as the input device 50, the touch panel may be provided on a display surface of the output device 40a.

Alternatively, the input device 50 is, for example, a device worn by a user, such as a glasses-type wearable device or a head mounted display (HMD), and various types of inputs are performed according to user's gesture or line-of-sight detected by these devices. Furthermore, the input device 50 includes a camera capable of detecting user's movement, and various types of inputs are performed according to user's gesture or line-of-sight detected from an image captured by the camera. Moreover, the input device 50 includes a microphone capable of collecting user's voice, and various types of inputs are performed by voice through the microphone. As described above, since the input device 50 is configured to be able to input various information in a contactless manner, a user who belongs to a clean area in particular can operate a device that belongs to a dirty area in a contactless manner. Furthermore, the user can operate the device without releasing his/her hand from a surgical instrument he/she holds, so that convenience of the user is improved.

### [3-2. Operation]

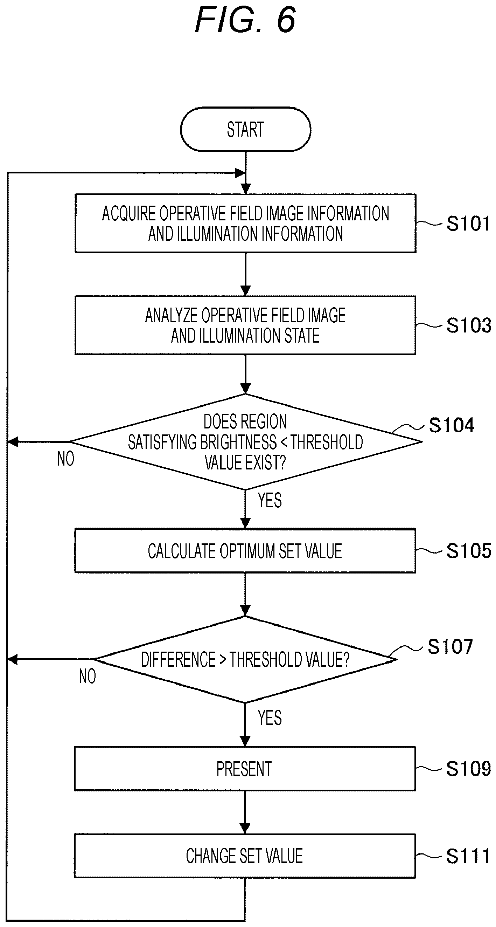

Next, operation of the surgical information processing device 10a according to the present embodiment will be described with reference to Fig. 6. Fig. 6 is a flowchart for explaining the operation of the surgical information processing device 10a according to the present embodiment. In the operation of the surgical information processing device 10a according to the present embodiment, a step of determining a dark part (S104) is added after the step of analyzing the operative field image and the illumination state (S103) in the flowchart shown in Fig. 3, and furthermore, a step of controlling the illumination device 30a (S111) is added after the step of performing presentation prompting the operation of the illumination device 30 (S109). Since S101 and S103 are similar to the operation of the surgical information processing device 10 according to the first embodiment, detailed description thereof will be omitted here.

In S104, the determination part 122a uses brightness distribution analyzed by the calculation part 121a in S103, and determines that a dark part exists in a case where there is a region where brightness is smaller than a predetermined threshold value on the operative field image (S104/ YES). In a case where it is determined that the dark part exists, the calculation part 121a calculates an optimum set value of the illumination device 30a on the basis of the acquired image information and the brightness distribution so that the dark part is irradiated with optimum light (S105). Specifically, the calculation part 121a calculates a set value that should be changed of the illumination device 30a by using a classifier generated by machine learning. Then, the determination part 122a compares a current set value of the illumination device 30a and the optimum set value of the illumination device 30a for the dark part, and determines whether or not to perform presentation prompting a user to change setting of the illumination device 30a (S107). Specifically, the determination part 122a compares the current set value of the illumination device 30a and the optimum set value of the illumination device 30a calculated by the calculation part 121, and in a case where a difference between the current set value and the optimum set value is larger than a predetermined threshold value, the determination part 122a determines that the setting of the illumination device 30a should be changed (S107/YES). The determination part 122a transmits a determination result to the presentation unit 130a.

Then, the presentation unit 130a receives the determination result from the determination part 122a. In a case where the setting of the illumination device 30a should be changed, the presentation unit 130a transmits a signal that prompts the user to operate the illumination device 30a to the output device 40a, and the output device 40a performs an output prompting the operation of the illumination device 30a (S109).

Thereafter, the user operates the input device 50 to change the set value of the illumination device 30a, and the input set value is transmitted to the control unit 150. Then, the control unit 150 performs control to change the set value of the illumination device 30a on the basis of the input set value (S111). The above S101 to S111 are repeated at any time. Therefore, the user can change the set value of the illumination device 30a on the spot and apply optimum illumination to the operative field while checking the presented operative field image and assist information. On the other hand, in S104, in a case where the determination part 122a determines that there is no region having brightness smaller than the predetermined threshold value, the operation is repeatedly repeated from S101 (S104/NO). Furthermore, in a case where the difference between the current set value of the illumination device 30a and the optimum set value of the illumination device 30a is equal to or less than the predetermined threshold value in S107, the determination part 122a does not present the operation, and the operation is repeatedly repeated from S101 (S107/NO).

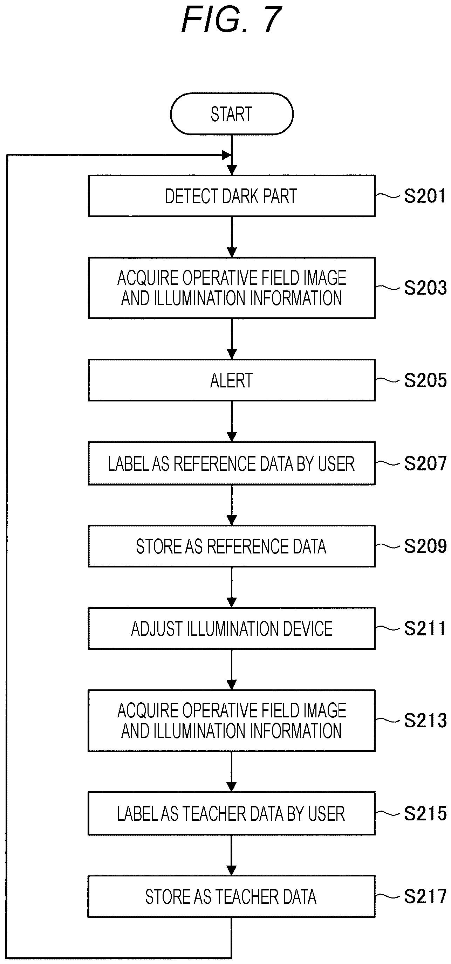

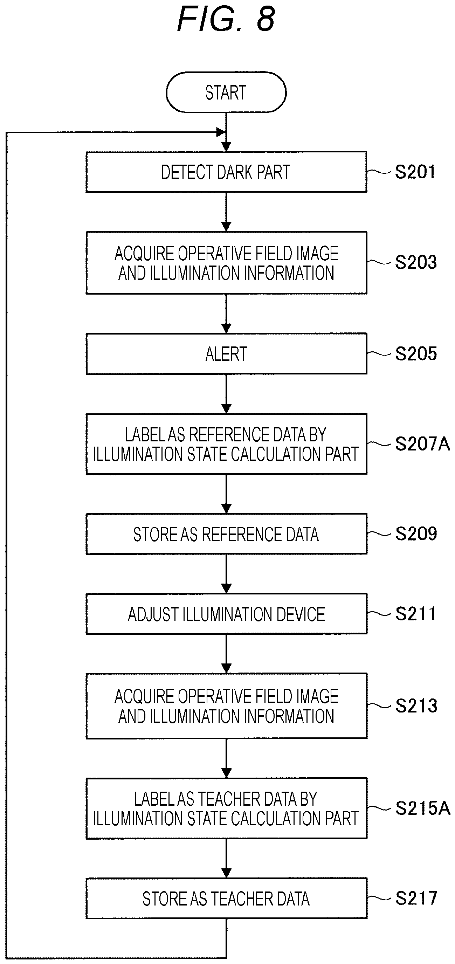

Here, a machine learning method for calculating an optimum set value of the illumination device 30a will be described in detail with reference to Figs. 7 and 8. Fig. 7 is a flowchart for explaining a flow of machine learning for calculating the optimum set value of the illumination device 30a in the surgical information processing device 10a according to the present embodiment. Fig. 8 is a flowchart for explaining another flow of machine learning for calculating the optimum set value of the illumination device 30a in the surgical information processing device 10a according to the present embodiment.

First, a machine learning method for generating a classifier used for calculating an optimum set value of the illumination device 30a by registering learning data by user's operation will be described with reference to Fig. 7.

The analysis unit 120a detects a dark part on an operative field image (S201). The detection of the dark part is performed in a flow similar to the flow of S101, S103, and S104 described above. Next, an operative field image, operative field image information, and a current set value of the illumination device 30a are acquired by the acquisition unit 110a (S203). The presentation unit 130a uses the output device 40a to transmit to the output device 40a a signal that prompts the user to operate the illumination device 30a. The output device 40a performs an output (alert) that prompts the operation of the illumination device 30a (S205). The user, for example, a perimeter nurse, labels and registers the operative field image, the operative field image information, and the current set value of the illumination device 30a acquired by the acquisition unit 110a as reference data (S207). The reference data is stored in the storage unit 140a (S209). Next, the user adjusts the illumination device 30a so that an operative field is suitable for surgery (S211). The adjusted operative field image, operative field image information, and set value of the illumination device 30a are acquired by the acquisition unit 110a (S213). The user labels and registers the adjusted operative field image, operative field image information, and set value of the illumination device 30a acquired by the acquisition unit 110a as teacher data (S215). The teacher data is stored in the storage unit 140a (S217). A classifier for calculating the optimum illumination state of the illumination device 30a is manufactured by repeating the above S201 to S217. Note that the alert in S205 may be omitted, and the surgical information processing device 10a may be set so that the output device 40a does not perform an output prompting the operation of the illumination device 30a. The user may operate the illumination device 30a with the alert off, collect an operative field image, operative field image information, and a current set value of the illumination device 30a, which will be reference data, and label and register the reference data. Furthermore, even in a case where the determination part 122a does not detect a dark part, when the user determines that there is a dark region in the operative field, the user may label and register an operative field image, operative field image information, and a current set value of the illumination device 30a at that time as reference data.

Next, a machine learning method for generating a classifier used for calculating an optimum set value of the illumination device 30a by registering learning data by the surgical information processing device 10a will be described with reference to Fig. 8. In the present method, a user does not label and register reference data and teacher data, but the surgical information processing device 10a collects learning data during surgery. Specifically, the procedure is performed as follows. The analysis unit 120a detects a dark part on an operative field image (S201). Next, an operative field image, operative field image information, and a current set value of the illumination device 30a are acquired by the acquisition unit 110a (S203). The presentation unit 130a uses the output device 40a to transmit to the output device 40a a signal that prompts the user to operate the illumination device 30. The output device 40a performs an output (alert) that prompts the operation of the illumination device 30 (S205). After the presentation unit 130a issues the alert by the output device 40a, the acquisition unit 110a labels and registers the operative field image, the operative field image information, and the current set value of the illumination device 30a as reference data (S207A). The reference data is stored in the storage unit 140 (S209). Subsequently, the user adjusts the illumination device 30 so that an operative field is suitable for surgery (S211). The adjusted operative field image, operative field image information, and set value of the illumination device 30a are acquired by the acquisition unit 110 (S213). Thereafter, if there is no change in the set value of the illumination device 30a for a predetermined time, the determination part 122a considers that the illumination device 30 has been adjusted to an optimum state, and the adjusted operative field image, operative field image information, and set value of the illumination device 30a are labeled and registered as teacher data (S215A). The teacher data is stored in the storage unit 140a (S217). A classifier for calculating the optimum set value of the illumination device 30a is manufactured by repeating the above S201 to S217.

As described above, the classifier is generated by performing machine learning on learning data in which an operative field image for learning and an optimum illumination state of the illumination device 30 in the operative field image for learning are associated with each other. The surgical information processing device 10a according to the present embodiment can calculate the optimum set value of the illumination device 30 by inputting the operative field image acquired by the camera 20 to this classifier. By calculating the optimum set value of the illumination device 30a by the above method, the presentation unit 130a can perform presentation to the user at a more appropriate timing, and furthermore, can present the optimum set value of the illumination device 30a with higher accuracy. Note that, in the above description, a case where the classifier is generated by machine learning by registering the learning data by the user's operation, or by machine learning by registering the learning data by the surgical information processing device 10a during the surgery has been described. However, the classifier may be generated by machine learning by registering learning data by the surgical information processing device 10a before surgery, or may be generated by machine learning by registering learning data by the other user's operation in advance.

### [3-3. First modified example]

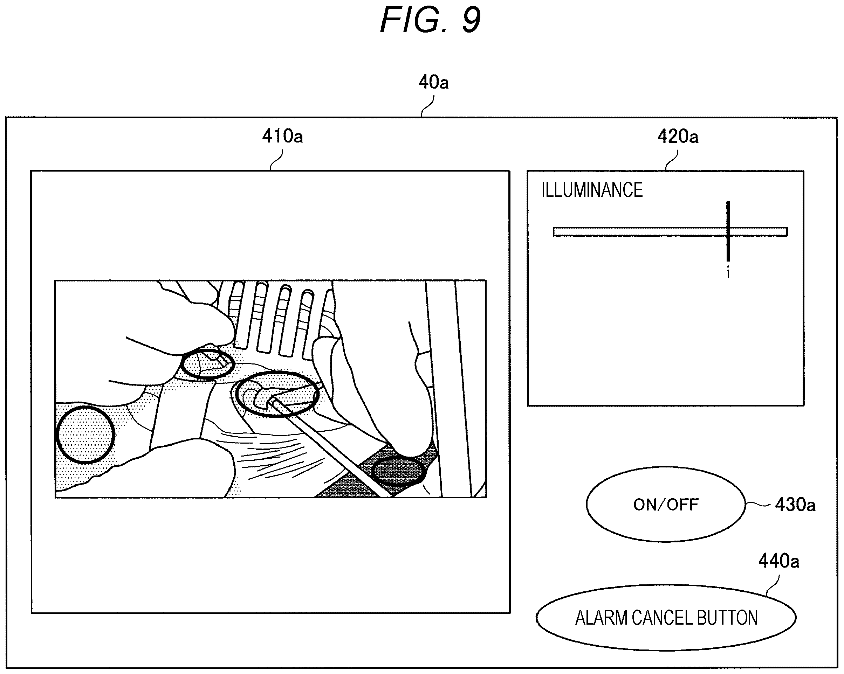

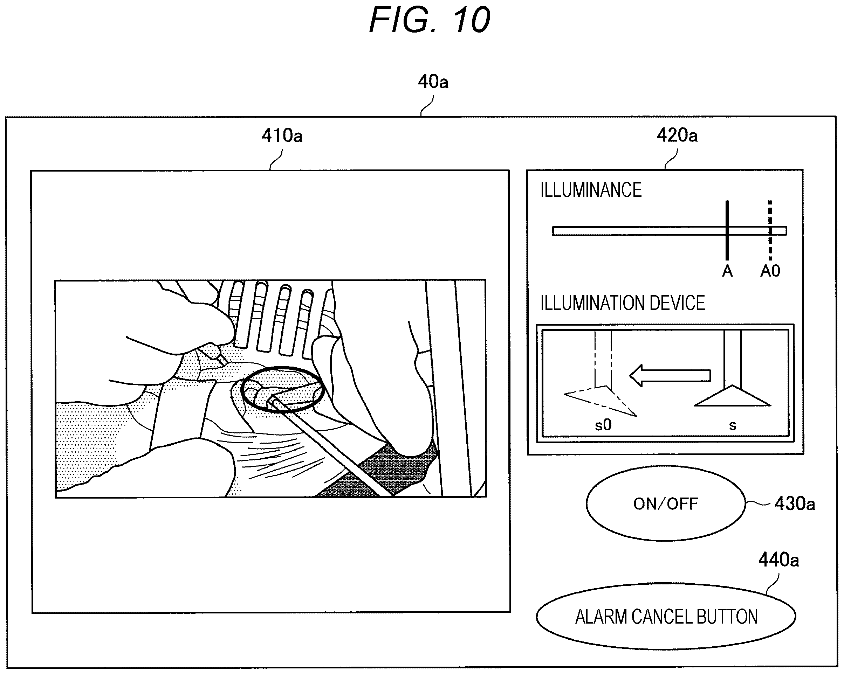

Next, a first modified example of the surgical information processing device 10a according to the second embodiment will be described with reference to Figs. 9 and 10. Fig. 9 is a schematic view for explaining a dark part presentation method according to the present embodiment. Fig. 10 is a schematic view for explaining the dark part presentation method according to the present embodiment.

As described above, the determination part 122a uses brightness distribution analyzed by the calculation part 121a, and determines that a dark part exists in a case where there is a region where brightness is smaller than a predetermined threshold value on an operative field image. In Fig. 5, a case where there is one dark region in the operative site is shown, but as shown in Fig. 9, the presentation unit 130a may present a plurality of dark parts in a region displayed in an operative field image by using the output device 40a. At this time, assist information presented by the presentation unit 130a by using the output device 40a may display a set value of the illumination device 30a for any one of the plurality of dark parts. Of the plurality of dark parts, a dark part whose brightness a user wants to adjust may be selected by operating the input device 50 by the user. For example, in a case where a touch panel is used as the input device 50, the dark part may be selected by the user touching the dark part that he/she wants to brighten. Furthermore, the user can also select a region other than the dark part presented by the presentation unit 130a and set the selected position as a position where brightness should be adjusted. Then, the analysis unit 120a calculates an optimum set value of the illumination device 30a for the selected dark part by the machine learning described above. As a result, for example, as shown in Fig. 10, the output device 40a superimposes and displays a display showing the selected dark part on a real-time image displayed in the operative field image display area 410a. Furthermore, the assist information display area 420a displays real-time illuminance A and illuminance A0 that is the optimum set value for the dark part shown in the operative field image display area 410a. Furthermore, the assist information display area 420a displays real-time arrangement S of the illumination device 30 and arrangement S0 of the illumination device 30 in an optimum illumination state. As described above, by presenting the plurality of dark parts generated in the operative field, the user can also adjust brightness of a portion that the user wants to watch other than an operative site where surgery is being performed, and can quickly determine the presence or absence of treatment for the portion. Furthermore, by selecting the portion other than the presented dark part by the user, the brightness of the portion that the user wants to watch can be quickly adjusted, and surgery time can be shortened.

### [3-4. Second modified example]

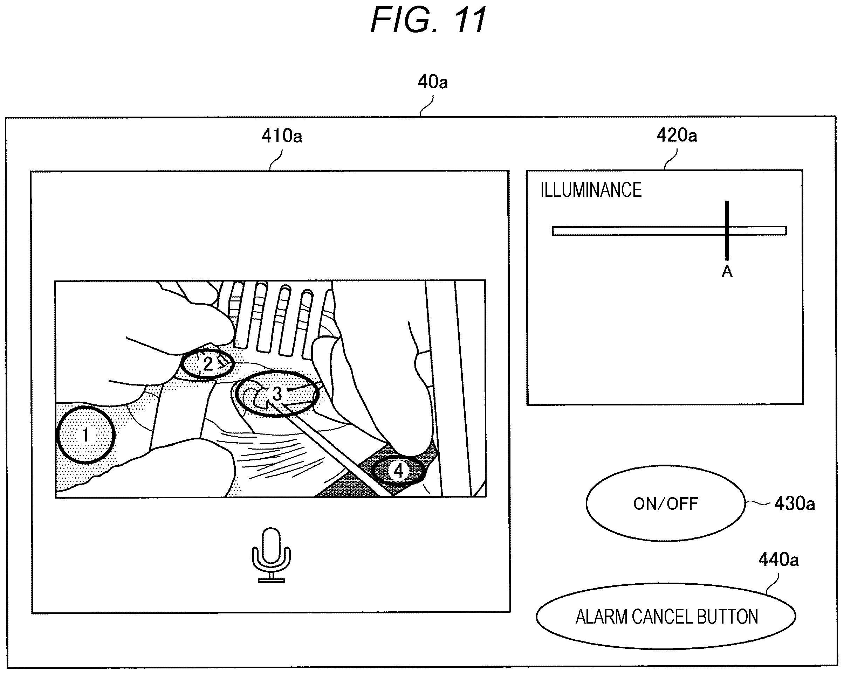

Next, a second modified example of the surgical information processing device 10a according to the second embodiment will be described with reference to Fig. 11. Fig. 11 is a schematic view for explaining a dark part presentation method according to the present modified example.

In the present modified example, as shown in Fig. 11, a plurality of dark parts in a region displayed in an operative field image is presented by using the output device 40a, but the presentation unit 130a may present a plurality of detected dark parts in a selectable manner. In Fig. 11, a number is assigned to each of the plurality of detected dark parts. For example, in a case where a microphone that recognizes a user's voice is used as the input device 50, a dark part to be brightened may be selected by reading the number assigned to the dark part by the user. Furthermore, for example, the microphone as the input device 50 recognizes the user's voice, and thus the illumination device 30a may be adjusted to have an optimum set value. Since the selection of the dark part and the adjustment of the illumination device 30a are performed by the voice, even in a case where the illumination device 30 or the input device 50 is not sterilized, a user who cannot touch an unsterilized unclean area, for example, an operator, can operate the illumination device 30a to realize an optimum illumination state.

### [3-5. Third modified example]

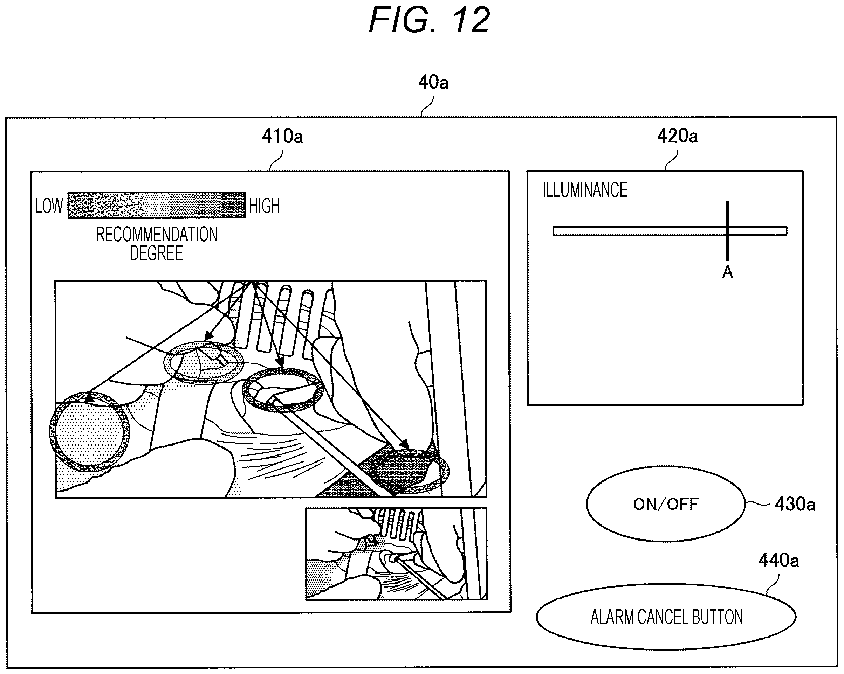

Next, a third modified example of the surgical information processing device 10a according to the third embodiment will be described with reference to Fig. 12. Fig. 12 is a schematic view for explaining a dark part presentation method according to the present modified example.

In the first modified example, as shown in Fig. 9, the presentation unit 130a presents the plurality of dark parts in the region displayed in the operative field image by using the output device 40a. However, as shown in Fig. 12, the presentation unit 130a may present a plurality of detected dark parts by changing a dark part display method according to a recommendation degree of a dark part whose brightness should be adjusted. For example, in Fig. 12, the dark part display method is changed and presented according to the recommendation degree of the dark part whose brightness should be adjusted. The recommendation degree of the dark part whose brightness should be adjusted may be set according to a distance between a position of an operative site and a dark part calculated by the known image processing technique, for example. Specifically, the presentation unit 130a may present a display showing a high recommendation degree to the dark part close to the operative site by using the output device 40a. Furthermore, the recommendation degree of the dark part whose brightness should be adjusted may be set on the basis of a classifier generated by machine learning. For example, by applying a classifier that distinguishes a dark part and an object with low reflectance inserted in an operative field to the classifier generated by the machine learning, accuracy of dark part detection can be improved. The recommendation degree of the dark part whose brightness should be adjusted is calculated by the determination part 122a in the method previously described. Furthermore, the determination part 122a can set the recommendation degree of the dark part whose brightness should be adjusted on the basis of all information acquired by the acquisition unit 110a and all information that can be calculated by the analysis unit 120a on the basis of the information acquired by the acquisition unit 110a. Note that the recommendation degree of the dark part whose brightness should be adjusted is not limited to the display method as shown in Fig. 12. A numerical value indicating the recommendation degree may be displayed near each dark part on a real-time image, or the recommendation degree and a coordinate value of the dark part may be displayed in the assist information display area 420a as assist information. By such a presentation, a user can quickly recognize the dark part whose brightness should be adjusted.

Furthermore, as shown in a lower part of Fig. 12, the presentation unit 130 may perform picture in picture (PinP) presentation of images before and after the dark part is detected as moving images. By presenting the moving images before and after the dark part is detected by the presentation unit 130, the user can specify a cause of the dark part.

### <4. Third embodiment>

### [4-1. Configuration]

Subsequently, a third embodiment according to the present disclosure will be described. A surgical information processing system 1b to which technology according to the present embodiment can be applied includes a surgical information processing device 10b according to the present embodiment, a camera 20b, an illumination device 30b, an output device 40b, and an input device 50b. Since the camera 20b and the illumination device 30b are similar to those described in the first embodiment and the output device 40b and the input device 50b are similar to those described in the second embodiment, detailed description thereof will be omitted here. The surgical information processing device 10b according to the present embodiment can perform various processing while mutually cooperating with the camera 20b, the illumination device 30b, the output device 40b, and the input device 50b in the surgical information processing system 1b. Furthermore, the surgical information processing device 10b according to the present embodiment can also carry out various processing while mutually cooperating with those having functions similar to camera 20b, the illumination device 30b, the output device 40b, and the input device 50b, without cooperating with the camera 20b, the illumination device 30b, the output device 40b, and the input device 50b in the surgical information processing system 1b.

The surgical information processing device 10b according to the present embodiment includes an acquisition unit 110b, an analysis unit 120b, a presentation unit 130b, a storage unit 140b, and a control unit 150b. Since the acquisition unit 110b, the storage unit 140b, and the control unit 150b are similar to those described in the second embodiment, detailed description thereof will be omitted here.

The analysis unit 120b further has a function of generating simulation image information for generating a simulation image that simulates an operative field in a case where a set value of the illumination device 30b has been changed, in addition to the function of the analysis unit 120a according to the second embodiment. A plurality of pieces of simulation image information may be generated according to a set value to be adjusted of the illumination device 30b. The set value to be adjusted of the illumination device 30b may be determined by machine learning so that brightness is suitable for a user to visually recognize an operative site.

The presentation unit 130b further has a function of presenting a simulation image of an operative field when a set value of the illumination device 30b has been changed, in addition to the function of the presentation unit 130a according to the second embodiment. A plurality of presented simulation images may be presented according to the simulation image information generated by the analysis unit 120b. Furthermore, a predetermined number of simulation images may be presented by a user.

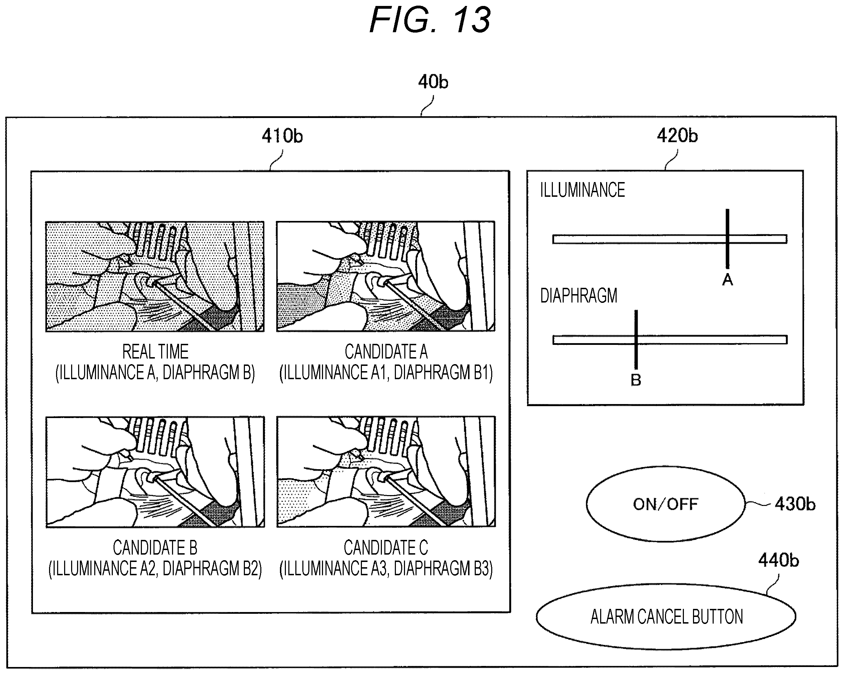

Here, information that the presentation unit 130b causes the output device 40b to output will be described with reference to Fig. 13. Fig. 13 is a schematic view for explaining a presented display according to the present embodiment. The output device 40b includes an operative field image display area 410b, an assist information display area 420b, a switch 430, and an alarm cancel button 440b. The switch 430b has a function of controlling ON/OFF of the illumination device 30, and the alarm cancel button 440b has a function of canceling an alarm issued at the time of prompting a user to set the illumination device 30b.

The operative field image display area 410b displays, for example, a current image acquired by the camera 20b and a simulation image of an operative field obtained when a set value of the illumination device 30b has been changed. Furthermore, in the assist information display area 420b, illuminance A of the illumination device 30b and a diaphragm B of the illumination device 30b are displayed for the current image. Specifically, in the operative field image display area 410b, one real-time operative field image and three simulation images of the operative field are presented in Fig. 13. These four images correspond to the image information transmitted from the presentation unit 130a. The presented real-time operative field image is a real-time operative field image I illuminated with the illuminance of the illumination device 30b adjusted to A and the diaphragm thereof adjusted to B. Furthermore, the presentation unit 130b presents an operative field image in a case where the illuminance of the illumination device 30b is adjusted to A1 and the diaphragm thereof is adjusted to B1 as a candidate A, an operative field image in a case where the illuminance of the illumination device 30b is adjusted to A2 and the diaphragm thereof is adjusted to B2 as a candidate B, and an operative field image in a case where the illuminance of the illumination device 30b is adjusted to A3 and the diaphragm thereof is adjusted to B3 as a candidate C. Then, one operative field image is selected from the above three candidates for the operative field image by operating the input device 50b by a user, and the illumination device 30b is adjusted to have illuminance and a diaphragm of a candidate image by the control unit 150b. By presenting the simulation images as the candidate images, the surgical information processing device 10b according to the present modified example can provide brightness of the operative field according to the operator.

Note that, of the four images, the real-time operative field image may be displayed relatively large as a main image, and the remaining three simulation images may be displayed relatively small as sub images.

### [4-2. Operation]

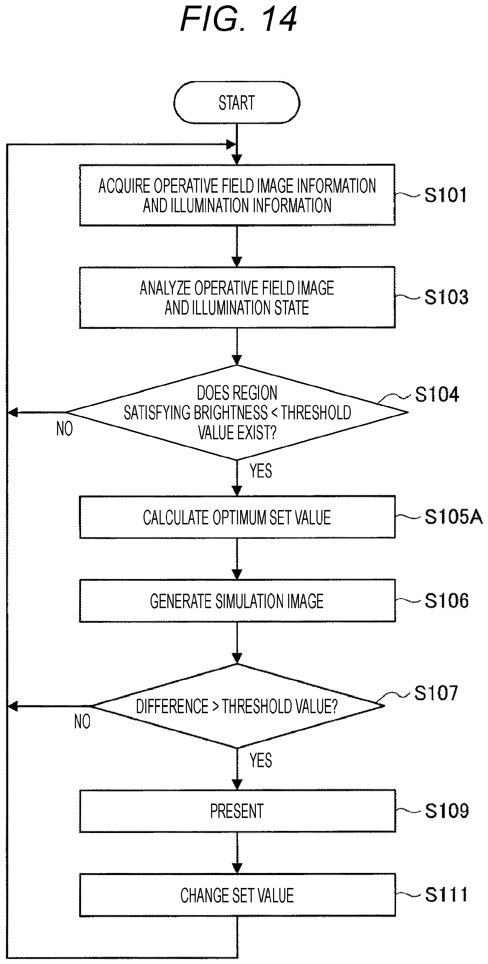

Next, operation of the surgical information processing device 10b according to the present embodiment will be described with reference to Fig. 14. Fig. 14 is a flowchart for explaining the operation of the surgical information processing device 10b according to the present embodiment. The operation of the surgical information processing device 10b according to the present embodiment is performed by adding a step of generating simulation image information (S106) after the step of calculating the optimum set value (S105A) in the flowchart shown in Fig. 6. In a flow of the operation of the surgical information processing device 10b according to the present embodiment, since S101 to S104 and S107 to S111 are similar to the operation of the surgical information processing device 10a according to the second embodiment, detailed description thereof is omitted here.

In S105A, the calculation part 121b calculates a plurality of optimum set values of the illumination device 30 on the basis of the acquired image information and the brightness distribution so that a dark part is irradiated with optimum light. Specifically, the calculation part 121b analyzes an operative field image to calculate an illumination state of an operative site, and calculates a plurality of set values to be changed, by using a classifier generated by machine learning.

Next, the analysis unit 120b generates a plurality of pieces of simulation image information of the operative field on the basis of the plurality of calculated optimum set values of the illumination device 30b (S106). As the simulation image information generated at this time, for example, images in which brightness of the operative field is the same and colors are different may be generated.

Thereafter, the determination part 122b included in the analysis unit 120b compares the set value of the illumination device 30b at the time when the operative field image has been acquired and the plurality of optimum set values of the illumination device 30b, and determines whether or not to perform presentation prompting a user to change setting of the illumination device 30b (S107). Specifically, the determination part 122b compares a current set value of the illumination device 30b and the optimum set value of the illumination device 30b calculated by the calculation part 121b, and in a case where a difference between the current set value and the optimum set value is larger than a predetermined threshold, the determination part 122b determines that the setting of the illumination device 30b should be changed (S107/YES). In a case where the determination part 122b determines that the setting of the illumination device 30b should be changed, the analysis unit 120b transmits the generated simulation image information to the presentation unit 130b. On the other hand, in S107, in a case where the difference between the current set value and the optimum set value of the illumination device 30b is equal to or smaller than the predetermined threshold value, the operation is repeatedly repeated from S101 (S107/NO).

Next, the output device 40b performs an output prompting the operation of the illumination device 30b (S109). Here, a real-time operative field image and a plurality of simulation images based on the plurality of pieces of simulation image information generated by the analysis unit 120b are presented. For example, the output device 40b displays one real-time operative field image and three simulation images in Fig. 13. One of the displayed operative field images is a real-time operative field image illuminated with illuminance of the illumination device 30b adjusted to A and a diaphragm thereof adjusted to B. Furthermore, the presentation unit 130b presents an operative field image in a case where the illuminance of the illumination device 30b is adjusted to A1 and the diaphragm thereof is adjusted to B1 as a candidate A, an operative field image in a case where the illuminance of the illumination device 30b is adjusted to A2 and the diaphragm thereof is adjusted to B2 as a candidate B, and an operative field image in a case where the illuminance of the illumination device 30b is adjusted to A3 and the diaphragm thereof is adjusted to B3 as a candidate C. Furthermore, the output device 40b displays the illuminance A and the diaphragm B of the illumination device 30b in real time as assist information.

Thereafter, one simulation image is selected from the above three candidates for the operative field image by operating the input device 50b by the user, and the image information of the selected simulation image is transmitted to the control unit 150b. Then, the control unit 150b changes the set value of the illumination device 30b so that the operative field is illuminated according to the selected operative field image (S111). For example, in Fig. 13, in a case where the candidate 2 is selected by the user, the illuminance of the illumination device 30b is adjusted from the illuminance A to the illuminance A2, and the diaphragm is adjusted from the diaphragm B to the diaphragm B2. The above S101 to S111 are repeated at any time. Therefore, even in a case where the user is different for each surgery, it is possible to apply optimum illumination to the operative field according to the user.

### <5. Hardware configuration>

The embodiments according to the present disclosure have been described above. The image processing described above is realized by the cooperation of software and hardware of an information processing device described below.

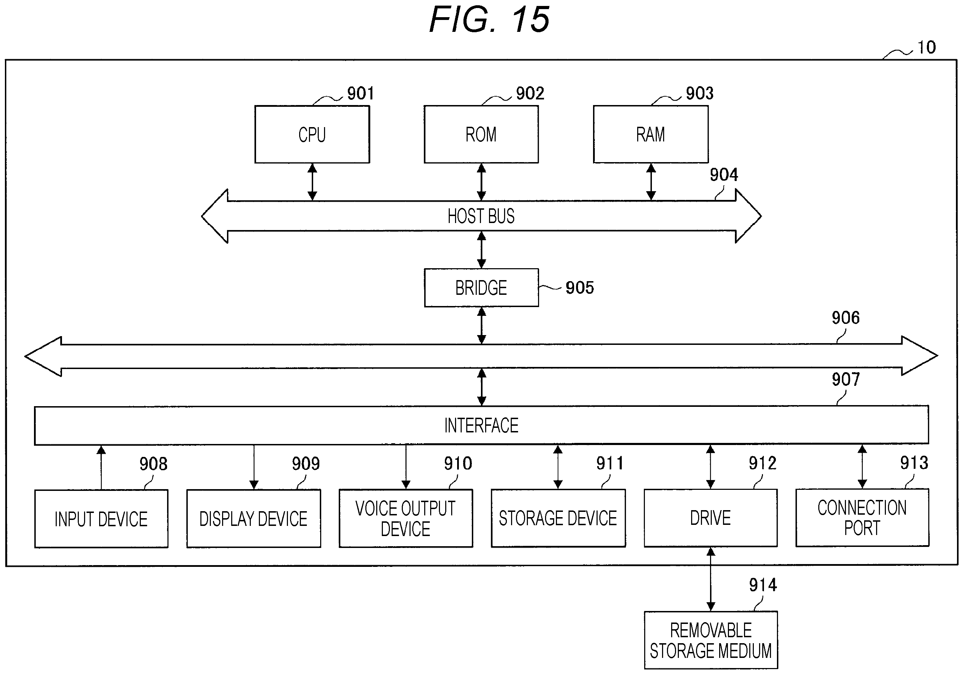

Next, a hardware configuration example of the surgical information processing device 10 according to one embodiment of the present disclosure will be described. Fig. 15 is a block diagram showing the hardware configuration example of the surgical information processing device 10 according to the one embodiment of the present disclosure. Referring to Fig. 15, the surgical information processing device 10 includes, for example, a CPU 901, a ROM 902, a RAM 903, a host bus 904, a bridge 905, an external bus 906, an interface 907, an input device 908, a display device. 909, a voice output device 910, a storage device 911, a drive 912, a connection port 913, and a removable storage medium 914. Note that the hardware configuration shown here is an example, and some of the components may be omitted. Furthermore, the surgical information processing device 10 may further include components other than the components shown here.

The CPU 901 functions as, for example, an arithmetic processing device or a control device, and controls all or part of operation of the components on the basis of various programs recorded in the ROM 902, the RAM 903, the storage device 911, or the removable storage medium 914.

The ROM 902 is means for storing a program read into the CPU 901, data used for calculation, and the like. The RAM 903 temporarily or permanently stores, for example, a program read into the CPU 901 and various parameters and the like that change appropriately when the program is executed.

The CPU 901, the ROM 902, and the RAM 903 are connected to each other, for example, via the host bus 904 capable of high-speed data transmission. On the other hand, the host bus 904 is connected to, for example, the external bus 906 having a relatively low data transmission rate via the bridge 905. Furthermore, the external bus 906 is connected with various components via the interface 907.

For example, a mouse, a keyboard, a touch panel, a button, a switch, a lever, and the like can be used as the input device 908. Moreover, a remote controller capable of transmitting a control signal using infrared rays or other radio waves may be used as the input device 908. Furthermore, the input device 908 includes a voice input device such as a microphone. In the surgical information processing device 10 according to the one embodiment of the present disclosure, the input device 908 corresponds to the input device 50 described above.

The display device 909 is, for example, a display device such as a cathode ray tube (CRT), an LCD, or an organic EL, a printer, and the like, and the voice output device 910 is an audio output device such as a speaker and a headphone, and the like. Both of the display device 909 and the voice output device 910 are devices that can visually or audibly notify a user of acquired information.

The storage device 911 is a device for storing various data. For example, a magnetic storage device such as a hard disk drive (HDD), a semiconductor storage device, an optical storage device, a magneto-optical storage device, or the like is used as the storage device 911.

The drive 912 is, for example, a device that reads information recorded in the removable storage medium 914 such as a magnetic disk, an optical disk, a magneto-optical disk, or a semiconductor memory, or writes information in the removable storage medium 914.

The removable storage medium 914 is, for example, a DVD medium, a Blu-ray (registered trademark) medium, an HD DVD medium, various semiconductor storage media, and the like. Of course, the removable storage medium 914 may be, for example, an IC card equipped with a noncontact type IC chip, an electronic device, or the like.

The connection port 913 is, for example, a port for connecting an external connection device such as a universal serial bus (USB) port, an IEEE1394 port, a small computer system interface (SCSI), an RS-232C port, or an optical audio terminal.

### <6. Conclusion>

As described above, according to the present disclosure, in a case where setting of the illumination device should be changed, presentation that prompts operation of the illumination device 30 is performed. As a result, a user can quickly understand necessity of adjusting an illumination state in an operative field.

The preferred embodiments of the present disclosure have been described above in detail with reference to the accompanying drawings, but the technical scope of the present disclosure is not limited to such examples. It is obvious that a person having ordinary knowledge in the technical field of the present disclosure can come up with various changes or modifications within the scope of the technical idea described in the claims.

Furthermore, the effects described in the present specification are merely explanatory or exemplifying ones, and are not limiting. In other words, the technology according to the present disclosure can exert other effects that are apparent to those skilled in the art from the description of the present specification, in addition to or instead of the above effects.

Furthermore, the processing described by using the flowcharts in the present specification does not necessarily have to be executed in the illustrated order. Some processing steps may be performed in parallel. Furthermore, additional processing steps may be adopted, and some processing steps may be omitted.

### REFERENCE SIGNS LIST

1, 1a, 1b Surgical information processing system
10, 10a, 10b Surgical information processing device
20, 20a, 20b Camera
30, 30a, 30b Illumination device
40, 40a, 40b Output device
50, 50b Input device
110, 110a, 110b Acquisition unit
111, 111a, 111b Image information acquisition part
112, 112a, 112b Illumination information acquisition part
120, 120a, 120b Analysis unit
121, 121a, 121b Calculation part
122, 122a, 122b Determination part
130, 130a, 130b Presentation unit
140, 140a, 140b Storage unit
150, 150b Control unit

## Claims

1. A surgical information processing device (10) comprising:
an acquisition unit (110) configured to acquire an operative field image including an operative field illuminated by an illumination device (30) and first illumination information indicating an illumination state of the illumination device;
a presentation unit (130) configured to perform a presentation based on the operative field image and the first illumination information acquired by the acquisition unit to a user; and
an analysis unit (120) configured to calculate second illumination information indicating an ideal illumination state of the illumination device by machine learning from the operative field image,
wherein the presentation unit is configured to perform the presentation to the user on a basis of a result of a comparison between the first illumination information and the second illumination information.

2. The surgical information processing device according to claim 1,
wherein the analysis unit is configured to calculate the second illumination information by inputting the operative field image into a classifier, the classifier being generated by performing machine learning in advance on learning data in which the operative field image for learning and the ideal illumination state in the operative field image for the learning are associated with each other.

3. The surgical information processing device according to any of claims 1 and 2,
wherein in a case where a dark part having a brightness equal to or less than a predetermined threshold value occurs in the operative field image, the presentation unit is configured to perform the presentation based on the operative field image and the first illumination information to the user.

4. The surgical information processing device according to claim 3,
wherein the presentation unit is configured to present a plurality of the dark parts in a selectable manner, and presents second illumination information for the dark part selected by the user.

5. The surgical information processing device according to claim 4,
wherein the presentation unit is configured to select the dark part on a basis of a voice uttered by the user.

6. The surgical information processing device according to any of claims 3 to 5,
wherein the presentation unit is configured to change a display indicating a position of the dark part and displays it by an external output device according to the position of the dark part in the operative field image.

7. The surgical information processing device according to any of claims 3 to 6,
wherein the presentation unit is configured to present images before and after the time when it is determined that the dark part has occurred.

8. The surgical information processing device according to any of claims 1 to 7,
wherein the analysis unit is configured to calculate the second illumination information about a position specified by the user in the operative field.

9. The surgical information processing device according to any of claims 1 to 8,
wherein the analysis unit is configured to calculate a difference between the first illumination information and the second illumination information, and
the presentation unit is configured to perform the presentation to the user when the difference exceeds a predetermined threshold value.

10. The surgical information processing device according to claim 9,
wherein the presentation unit is configured to output a signal that evokes operation of the illumination device when the difference exceeds the threshold value.

11. The surgical information processing device according to any of claims 1 to 10,
wherein the presentation unit is configured to present the first illumination information when the operative field image has been acquired in a predetermined presentation method.

12. The surgical information processing device according to any of claims 1 to 11,
wherein the analysis unit is configured to calculate the econd illumination information indicating an ideal illumination state of the illumination device for a certain region by machine learning from the operative field image, and
the acquisition unit is configured to acquire a region for calculating the second illumination information by detecting a surgical instrument in the operative field, a line-of-sight of the user or a different user.

13. An information processing method comprising:
by a processor,
acquiring (S101) an operative field image including an operative field illuminated by an illumination device (30) and first illumination information indicating an illumination state of the illumination device;
performing (S109) a presentation based on the acquired operative field image and the acquired first llumination information to a user; and
calculating (S105) second illumination information indicating an ideal illumination state of the illumination device by machine learning from the operative field image;
wherein performing (S109) the presentation to the user is on a basis of a result of a comparison between the first illumination information and the second illumination information.

14. A program to allow a computer to function as:
an acquisition unit (110) that acquires an operative field image including an operative field illuminated by an illumination device (30) and first illumination information indicating an illumination state of the illumination device;
a presentation unit (130) that performs a presentation based on the operative field image and the first illumination information acquired by the acquisition unit to a user; and
an analysis unit (120) that calculates second illumination information indicating an ideal illumination state of the illumination device by machine learning from the operative field image;
wherein the presentation unit performs presentation to the user on a basis of a result of a comparison between the first illumination information and the second illumination information.

## Patentansprüche

1. Chirurgische Informationsverarbeitungsvorrichtung (10), umfassend:
eine Erfassungseinheit (110), die dafür ausgelegt ist, ein Bild eines Operationsfeldes zu erfassen, das ein Operationsfeld, welches durch eine Beleuchtungsvorrichtung (30) beleuchtet ist, und erste Beleuchtungsinformationen, die einen Beleuchtungszustand der Beleuchtungsvorrichtung anzeigen, beinhaltet;
eine Präsentationseinheit (130), die dafür ausgelegt ist, eine Präsentation für einen Benutzer durchzuführen, basierend auf dem Bild des Operationsfeldes und den von der Erfassungseinheit erfassten ersten Beleuchtungsinformationen; und
eine Analyseeinheit (120), die dafür ausgelegt ist, zweite Beleuchtungsinformationen, die einen idealen Beleuchtungszustand der Beleuchtungsvorrichtung anzeigen, durch maschinelles Lernen aus dem Bild des Operationsfeldes zu berechnen,
wobei die Präsentationseinheit dafür ausgelegt ist, die Präsentation für den Benutzer basierend auf einem Ergebnis eines Vergleichs zwischen den ersten Beleuchtungsinformationen und den zweiten Beleuchtungsinformationen durchzuführen.

2. Chirurgische Informationsverarbeitungsvorrichtung gemäß Anspruch 1,
wobei die Analyseeinheit dafür ausgelegt ist, die zweiten Beleuchtungsinformationen zu berechnen, indem das Bild des Operationsfeldes in einen Klassifikator eingegeben wird, wobei der Klassifikator durch maschinelles Lernen im Voraus anhand von Lerndaten erzeugt wird, in denen das Bild des Operationsfeldes für das Lernen und der ideale Beleuchtungszustand in dem Bild des Operationsfeldes für das Lernen miteinander verknüpft sind.

3. Chirurgische Informationsverarbeitungsvorrichtung gemäß Anspruch 1 oder 2,
wobei in einem Fall, in dem ein dunkler Teil mit einer Helligkeit gleich oder kleiner als ein vorbestimmter Schwellenwert in dem Bild des Operationsfeldes auftritt, die Präsentationseinheit dafür ausgelegt ist, die Präsentation basierend auf dem Bild des Operationsfeldes und den ersten Beleuchtungsinformationen für den Benutzer durchzuführen.

4. Chirurgische Informationsverarbeitungsvorrichtung gemäß Anspruch 3,
wobei die Präsentationseinheit dafür ausgelegt ist, mehrere der dunklen Teile in einer auswählbaren Weise zu präsentieren, und zweite Beleuchtungsinformationen für den vom Benutzer ausgewählten dunklen Teil präsentiert.

5. Chirurgische Informationsverarbeitungsvorrichtung gemäß Anspruch 4,
wobei die Präsentationseinheit dafür ausgelegt ist, den dunklen Teil basierend auf einer stimmlichen Äußerung des Benutzers auszuwählen.

6. Chirurgische Informationsverarbeitungsvorrichtung gemäß einem der Ansprüche 3 bis 5,
wobei die Präsentationseinheit dafür ausgelegt ist, eine Anzeige, die eine Position des dunklen Teils anzeigt, zu ändern, und diese durch eine externe Ausgabevorrichtung anzeigt, gemäß der Position des dunklen Teils im Bild des Operationsfeldes.

7. Chirurgische Informationsverarbeitungsvorrichtung gemäß einem der Ansprüche 3 bis 6,
wobei die Präsentationseinheit dafür ausgelegt ist, Bilder vor und nach dem Zeitpunkt, zu dem bestimmt wird, dass der dunkle Teil aufgetreten ist, zu präsentieren.

8. Chirurgische Informationsverarbeitungsvorrichtung gemäß einem der Ansprüche 1 bis 7,
wobei die Analyseeinheit dafür ausgelegt ist, die zweiten Beleuchtungsinformationen über eine vom Benutzer angegebene Position im Operationsfeld zu berechnen.

9. Chirurgische Informationsverarbeitungsvorrichtung gemäß einem der Ansprüche 1 bis 8,
wobei die Analyseeinheit dafür ausgelegt ist, eine Differenz zwischen den ersten Beleuchtungsinformationen und den zweiten Beleuchtungsinformationen zu berechnen, und
wobei die Präsentationseinheit dafür ausgelegt ist, die Präsentation für den Benutzer durchzuführen, wenn die Differenz einen vorbestimmten Schwellenwert überschreitet.

10. Chirurgische Informationsverarbeitungsvorrichtung gemäß Anspruch 9,
wobei die Präsentationseinheit dafür ausgelegt ist, ein Signal auszugeben, das den Betrieb der Beleuchtungsvorrichtung auslöst, wenn die Differenz den Schwellenwert überschreitet.

11. Chirurgische Informationsverarbeitungsvorrichtung gemäß einem der Ansprüche 1 bis 10,
wobei die Präsentationseinheit dafür ausgelegt ist, die ersten Beleuchtungsinformationen zu präsentieren, wenn das Bild des Operationsfeldes nach einem vorbestimmten Präsentationsverfahren erfasst worden ist.

12. Chirurgische Informationsverarbeitungsvorrichtung gemäß einem der Ansprüche 1 bis 11,
wobei die Analyseeinheit dafür ausgelegt ist, die zweiten Beleuchtungsinformationen, die einen idealen Beleuchtungszustand der Beleuchtungsvorrichtung für einen bestimmten Bereich anzeigen, durch maschinelles Lernen aus dem Bild des Operationsfeldes zu berechnen, und
wobei die Erfassungseinheit dafür ausgelegt ist, einen Bereich zum Berechnen der zweiten Beleuchtungsinformationen zu erfassen, indem ein chirurgisches Instrument im Operationsfeld, eine Sichtlinie des Benutzers oder ein anderer Benutzer erkannt wird.

13. Informationsverarbeitungsverfahren, umfassend:
durch einen Prozessor, Erfassen (S101) eines Bildes des Operationsfeldes, das ein Operationsfeld, welches durch eine Beleuchtungsvorrichtung (30) beleuchtet ist, und erste Beleuchtungsinformationen, die einen Beleuchtungszustand der Beleuchtungsvorrichtung anzeigen, beinhaltet;
Durchführen (S109) einer Präsentation basierend auf dem erfassten Bild des Operationsfeldes und den erfassten ersten Beleuchtungsinformationen für einen Benutzer; und
Berechnen (S105) zweiter Beleuchtungsinformationen, die einen idealen Beleuchtungszustand der Beleuchtungsvorrichtung anzeigen, durch maschinelles Lernen aus dem Bild des Operationsfeldes,
wobei das Durchführen (S109) der Präsentation für den Benutzer basierend auf einem Ergebnis eines Vergleichs zwischen den ersten Beleuchtungsinformationen und den zweiten Beleuchtungsinformationen erfolgt.

14. Programm, um zu ermöglichen, dass ein Computer arbeitet als:
eine Erfassungseinheit (110), die ein Bild des Operationsfeldes erfasst, das ein Operationsfeld, welches durch eine Beleuchtungsvorrichtung (30) beleuchtet ist, und erste Beleuchtungsinformationen, die einen Beleuchtungszustand der Beleuchtungsvorrichtung anzeigen, beinhaltet;
eine Präsentationseinheit (130), die basierend auf dem Bild des Operationsfeldes und den von der Erfassungseinheit erfassten ersten Beleuchtungsinformationen eine Präsentation für einen Benutzer durchführt; und
eine Analyseeinheit (120), die zweite Beleuchtungsinformationen, die einen idealen Beleuchtungszustand der Beleuchtungsvorrichtung anzeigen, durch maschinelles Lernen aus dem Bild des Operationsfeldes berechnet;
wobei die Präsentationseinheit die Präsentation für den Benutzer basierend auf einem Ergebnis eines Vergleichs zwischen den ersten Beleuchtungsinformationen und den zweiten Beleuchtungsinformationen durchführt.

## Revendications

1. Dispositif de traitement d'informations chirurgicales (10) comprenant :
une unité d'acquisition (110) configurée pour acquérir une image de champ opératoire comprenant un champ opératoire éclairé par un dispositif d'éclairage (30) et des premières informations d'éclairage indiquant un état d'éclairage du dispositif d'éclairage ;
une unité de présentation (130) configurée pour effectuer une présentation à un utilisateur sur la base de l'image de champ opératoire et des premières informations d'éclairage acquises par l'unité d'acquisition ; et
une unité d'analyse (120) configurée pour calculer des secondes informations d'éclairage indiquant un état d'éclairage idéal du dispositif d'éclairage par apprentissage automatique à partir de l'image de champ opératoire,
où l'unité de présentation est configurée pour effectuer la présentation, à l'attention de l'utilisateur, sur la base d'un résultat d'une comparaison entre les premières informations d'éclairage et les secondes informations d'éclairage.

2. Dispositif de traitement d'informations chirurgicales selon la revendication 1,
dans lequel l'unité d'analyse est configurée pour calculer les secondes informations d'éclairage en entrant l'image de champ opératoire dans un classificateur, le classificateur étant généré en effectuant un apprentissage automatique à l'avance sur des données d'apprentissage où l'image de champ opératoire pour l'apprentissage et l'état d'éclairage idéal dans l'image de champ opératoire pour l'apprentissage sont associés l'un à l'autre.

3. Dispositif de traitement d'informations chirurgicales selon l'une quelconque des revendications 1 et 2,
dans lequel, dans un cas où une partie sombre ayant une luminosité égale ou inférieure à une valeur de seuil prédéterminée apparaît dans l'image de champ opératoire, l'unité de présentation est configurée pour effectuer la présentation à l'utilisateur sur la base de l'image de champ opératoire et des premières informations d'éclairage.

4. Dispositif de traitement d'informations chirurgicales selon la revendication 3,
dans lequel l'unité de présentation est configurée pour présenter une pluralité de parties sombres d'une manière sélectionnable, et présente des secondes informations d'éclairage pour la partie sombre sélectionnée par l'utilisateur.

5. Dispositif de traitement d'informations chirurgicales selon la revendication 4,
dans lequel l'unité de présentation est configurée pour sélectionner la partie sombre sur la base d'une voix prononcée par l'utilisateur.

6. Dispositif de traitement d'informations chirurgicales selon l'une quelconque des revendications 3 à 5,
dans lequel l'unité de présentation est configurée pour modifier un affichage indiquant une position de la partie sombre et l'affiche par un dispositif de sortie externe en fonction de la position de la partie sombre dans l'image du champ opératoire.

7. Dispositif de traitement d'informations chirurgicales selon l'une quelconque des revendications 3 à 6,
dans lequel l'unité de présentation est configurée pour présenter des images avant et après le moment où il est déterminé que la partie sombre est survenue.

8. Dispositif de traitement d'informations chirurgicales selon l'une quelconque des revendications 1 à 7,
dans lequel l'unité d'analyse est configurée pour calculer les secondes d'éclairage relatives à une position spécifiée par l'utilisateur dans le champ opératoire.

9. Dispositif de traitement d'informations chirurgicales selon l'une quelconque des revendications 1 à 8,
dans lequel l'unité d'analyse est configurée pour calculer une différence entre les premières informations d'éclairage et les secondes informations d'éclairage, et l'unité de présentation est configurée pour effectuer la présentation à l'utilisateur lorsque la différence dépasse une valeur seuil prédéterminée.

10. Dispositif de traitement d'informations chirurgicales selon la revendication 9,
dans lequel l'unité de présentation est configurée pour émettre un signal qui évoque le fonctionnement du dispositif d'éclairage lorsque la différence dépasse la valeur de seuil.

11. Dispositif de traitement d'informations chirurgicales selon l'une quelconque des revendications 1 à 10,
dans lequel l'unité de présentation est configurée pour présenter les premières informations d'éclairage lorsque l'image du champ opératoire a été acquise selon un procédé de présentation prédéterminé.

12. Dispositif de traitement d'informations chirurgicales selon l'une quelconque des revendications 1 à 11,
dans lequel l'unité d'analyse est configurée pour calculer les secondes informations d'éclairage indiquant un état d'éclairage idéal du dispositif d'éclairage pour une certaine région par apprentissage automatique à partir de l'image du champ opératoire, et
l'unité d'acquisition est configurée pour acquérir une région pour calculer les secondes informations d'éclairage en détectant un instrument chirurgical dans le champ opératoire, dans une ligne de visée de l'utilisateur ou celle d'un utilisateur différent.

13. Procédé de traitement d'informations comprenant les étapes suivantes exécutées par un processeur :
acquérir (S101) une image de champ opératoire comprenant un champ opératoire éclairé par un dispositif d'éclairage (30) et des premières informations d'éclairage indiquant un état d'éclairage du dispositif d'éclairage ;
effectuer (S109) une présentation à un utilisateur sur la base de l'image du champ opératoire acquise et des premières informations d'éclairage acquises ; et
calculer (S105) des secondes informations d'éclairage indiquant un état d'éclairage idéal du dispositif d'éclairage par apprentissage automatique à partir de l'image du champ opératoire ;
où l'exécution (S109) de la présentation à l'utilisateur s'effectue sur la base d'un résultat d'une comparaison entre les premières informations d'éclairage et les secondes informations d'éclairage.

14. Programme pour permettre à un ordinateur de fonctionner comme :
une unité d'acquisition (110) qui acquiert une image de champ opératoire comprenant un champ opératoire éclairé par un dispositif d'éclairage (30) et des premières informations d'éclairage indiquant un état d'éclairage du dispositif d'éclairage ;
une unité de présentation (130) qui effectue une présentation, à l'attention d'un utilisateur, sur la base de l'image du champ opératoire et les premières informations d'éclairage acquises par l'unité d'acquisition ; et
une unité d'analyse (120) qui calcule des secondes informations d'éclairage indiquant un état d'éclairage idéal du dispositif d'éclairage par apprentissage automatique à partir de l'image de champ opératoire ;
où l'unité de présentation effectue une présentation à l'attention de utilisateur sur la base d'un résultat d'une comparaison entre les premières informations d'éclairage et les secondes informations d'éclairage.
